# EUROPEAN PATENT SPECIFICATION

(11) **EP 0 747 335 B1**
(45) Date of publication and mention of the grant of the patent: **13.10.1999**
(21) Application number: 96109104.8
(22) Date of filing: 07.06.1996
(51) Int. Cl.: C07C 7/20, C07C 5/327, C07C 15/46

(54) **Polymerization inhibitor process**
Verfahren zur Polymerisierungshemmung
Procédé d'inhibition de polymérisation

(30) Priority: 07.06.1995 US 672231
(43) Date of publication of application: 11.12.1996
(73) Proprietor: FINA TECHNOLOGY, INC., Plano, Texas 75024 (US)
(72) Inventor: Butler, James R., Houston, Texas 77059 (US); Merrill, James T., Katy, Texas 77449 (US)
(74) Representative: Leyder, Francis

(56) References cited:
- US-A- 3 426 091
- US-A- 4 113 787

## Description

The present invention relates to the inhibition of polymerization in a material stream containing a polymerizable monomer component and more particularly involves the polymerization of monovinyl aromatic monomers such as styrene contained in the vent gas off of a dehydrogenation unit.

In the manufacture of a monovinyl aromatic monomer such as styrene from a chemical feedstock such as ethylbenzene, the monomer is manufactured by dehydrogenating the feedstock in a dehydrogenation or "dehydro" unit. For example, ethylbenzene ("EB") feedstock is converted into styrene by passing the EB through an EB dehydro unit which removes hydrogen atoms from the EB molecules to form styrene molecules. The gaseous side products of the chemical reaction containing mostly hydrogen are drawn off the EB dehydro unit under a vacuum as dehydro vent gas for additional processing.

The vent gas from the dehydro unit normally comprises hydrogen CO₂, CO, H₂O vapor, and hydrocarbon vapours. In one instance, the vent gas may be distilled to remove the "heavies" (EB and styrene) which are recycled to the EB dehydro unit and into the styrene product line, respectively, with the "lights", including the hydrogen, being burned for fuel value. Alternatively, the entire vent gas stream can be burned for fuel gas.

In another instance, the dehydro vent gas may be cycled into a phenylacetylene reduction system comprising one or more catalyst reactors through which styrene monomer containing phenylacetylene contaminant is passed over a suitable catalyst in order to reduce the phenylacetylene contaminants to styrene by reacting with the hydrogen contained in the vent gas.

In each instance above, the dehydro vent gas must be removed from the dehydro reactors, compressed and sent to another reactor, distillation unit, or burner. Regardless of its end use, the vent gas which is at reduced presure must be compressed to about 3.1 bars (45 PSI) in order to transport it to the next stage of the process.

Problems arise when trying to compress EB dehydro vent gas because of the styrene monomer content of the gas, styrene being a fairly reactive element and one which is quick to polymerize. Because of the heat of compression in the vent gas compressor, styrene monomer will polymerize readily on the internal parts and surfaces of the compressor, causing malfunction and poor efficiency in the compressor. If the compressor has shutdown, the polymer causes it to "freeze". The restart is difficult.

The conventional solution for preventing polymer build-up in the vent gas compressor has been continuous "wash" or "flush" of EB injected with the vent gas into the compressor. This has no significant beneficial effect on the polymerization of the monomer but it does physically dissolve the polymer off of the compressor components and allows the compressor to continue running. The resultant EB/polymer solution must then be processed to remove the EB from the polymerized monomer which is usually low-grade low molecular weight material, having little commercial value. The disadvantage of this method is that reprocessing the EB/polymer solutions is expensive. The low grade polymer which is produced adds to the plant tar and is a loss of valuable raw materials.

The present invention overcomes these deficiencies by providing a system and a process whereby an EB flush is not necessary to remove polymer accumulations from vent gas compressor components because the process prevents polymerization of the monomer in the vent gas compressor.

### SUMMARY OF THE INVENTION

The process and system disclosed and claimed herein utilizes a polymerization inhibitor that is injected into the EB dehydro vent gas upstream of the vent gas compressor to prevent polymer from forming inside the compressor.

### BRIEF DESCRIPTION OF THE DRAWINGS

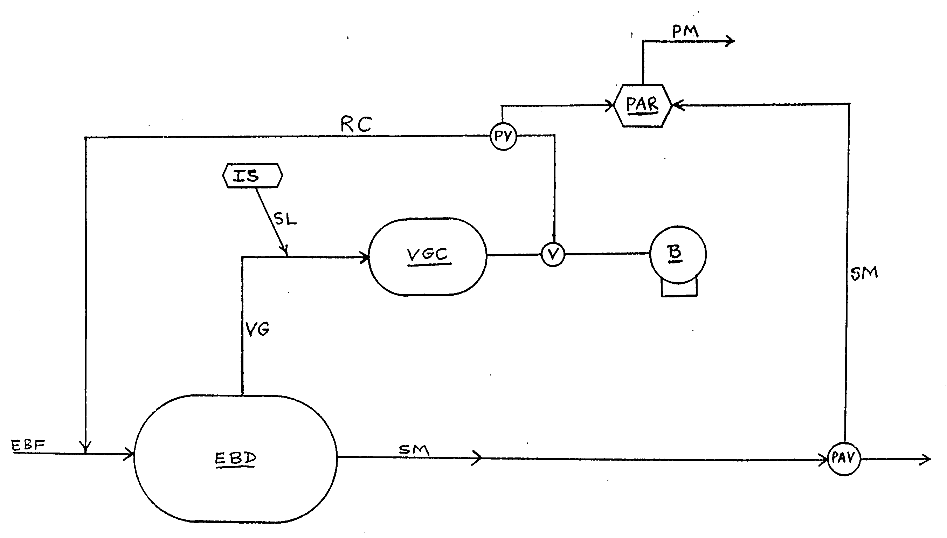

The figure is a schematic flow diagram of the system installed in a styrene manufacturing process.

### DESCRIPTION OF THE PREFERRED EMBODIMENTS

Referring to the figure, which is a schematic diagram of a styrene manufacturing process, a ethylbenzene feed stream EBF is fed to an ethylbenzene dehydrogenation reactor EBD. Hydrogen is removed from the ethylbenzene in the reactor EBD and a stream of styrene monomer SM is removed from the reactor. A by-product vent gas is drawn off of the reactor EBD through line VG and compressed in the vent gas compressor VGC. An inhibitor supply tank IS communicates with line VG through inhibitor supply line SL. A styrene inhibitor is injected through line SL into line VG upstream of the compressor VGC. The compressed vent gas/inhibitor mixture then passes through valve V and is either routed to a waste heat boiler B and burned as a fuel or alternatively, is routed through valve V through recycle line RC and injected back into the crude styrene from reactor EBD. Alternatively, a phenylacetylene removal system, PAR, may be utilized in conjunction with the present invention by the use of a second valve PV which recycles all or a portion of the compressed vent gas into a phenylacetylene reduction reactor PAR. Likewise, the styrene monomer feedstream from EBD is cycled through a phenylacetylene valve PAV and into the reactor PAR. The inhibited vent gas flowing through valve PV into the PAR reactor system is commingled or admixed with the styrene monomer stream and the hydrogen content of the vent gas reduces the phenylacetylene content of the styrene monomer into styrene which then is removed through purified monomer line PM.

In one alternative embodiment (not shown), valve PV can be replaced with a purification system such as a distillation unit to separate the heavier volatiles such as ethylbenzene and styrene flowing through valve V into recycle line RC to recycle these heavier volatiles back into the ethylbenzene feedstream EBF. The remainder of the vent gas minus the heavier volatiles is then cycled into the phenylacetylene reduction reactor PAR to further purify the styrene monomer stream from the reactor EBD.

The particular inhibitor used may be of any suitable styrene polymerization inhibitor which is capable of being injected into a stream of gas. For example, one particularly advantageous type of inhibitor was found to be that manufactured by Uniroyal Chemical Co. Inc. of Middleburg, Connecticut 06749, and designated as Uniroyal "Naugard", a family of phenylene/diamide styrene inhibitors which includes:
- Naugard I-2: N,N'[p-phenylene]bis[2-amino-5-methylhexane];
- Naugard I-3: N-[1,4-dimethylpentyl]-N'-phenyl-p-phenylene-diamine;
- Naugard I-4: 4-Isopropylamino diphenylamine;
- Naugard I-5: 4,6-dinitro-2-sec-butyl phenyl.
These and other inhibitors can be found more particularly described in U.S. Patent 4,664,845; 4,466,905; and 4,468,343, all of which are incorporated herein by reference. This inhibitor is injected into the vent gas stream through line SL upstream of the vent gas compressor and it is believed that such inhibitor will successfully prevent polymerization of styrene on the compressor components. It is also believed by the inventors that other similar injectable styrene inhibitors would also serve to prevent styrene polymerization on the internal compressor components. Although the process has not been actually attempted in an actual vent gas compressor, the inventors are of the belief that due to the nature of the inhibitor and the known characteristics of styrene monomer in vent gas applications, the presently disclosed inhibitor would sufficiently prevent polymerization in the compressor such that no polymer would be allowed to form therein.

## Claims

1. A process for dehydrogenating ethylbenzene to styrene, said process comprising:
passing an ethylbenzene feedstream over a dehydrogenation catalyst in a dehydrogenation reactor to form a product stream of styrene;
removing from said reactor a vent gas stream containing by-products, including hydrogen, ethylbenzene vapor, styrene, benzene and toluene vapors, CO, CO₂ and water vapor
injecting into said vent gas stream a styrene polymerization inhibitor; and, compressing said vent gas stream for further processing.

2. The process of Claim 1, wherein said polymerization inhibitor is a phenylenediamine compound.

3. A system for dehydrogenating ethylbenzene into styrene, said system comprising:
a catalytic dehydrogenation reactor adapted for dehydrogenating ethylbenzene into styrene
an ethylbenzene feedstream supply connected to said reactor;
a vent gas removal sub-system connected to said reactor and arranged to remove from said reactor vent gas by-products of ethylbenzene dehydrogenation;
a vent gas compressor connected with said removal sub-system and arranged to receive vent gas therefrom; and;
a polymerization inhibitor sub-system between said removal sub-system and said compressor arranged to inject a polymerization inhibitor into vent gas from said reactor.

## Patentansprüche

1. Verfahren für Dehydrieren von Ethylbenzol zu Styrol, wobei das Verfahren umfaßt:
Leiten eines Ethylbenzolzufuhrstromes über einen Dehydrierungskatalysator in einem Dehydrierungsreaktor unter Bilden eines Produktstroffes von Styrol, Entfernen eines Abgasstromes, enthaltend Nebenprodukte einschließlich Wasserstoff, Ethylbenzoldampf, Styrol-, Benzol- und Toluoldämpfe, CO, CO₂ und Wasserdampf aus dem Reaktor, Einspritzen eines Styrol-Polymerisationsinhibitors in den Abgasstrom und Komprimieren des Abgasstromes für weiteres verarbeiten.

2. Verfahren nach Anspruch 1, wobei der Polymerisationsinhibitor eine Phenylendiaminverbindung ist.

3. System für Dehydrieren von Ethylbenzol in Styrol, wobei das System umfaßt: einen für Dehydrieren von Ethylbenzol in Styrol angepaßten Reaktor für katalytische Dehydrierung, eine mit dem Reaktor verbundene Ethylbenzolzufuhrstromversorgung, ein Abgasentfernungsnebensystem, verbunden mit dem Reaktor und angeordnet, Abgasnebenprodukte von Ethylbenzoldehydrierung aus dem Reaktor zu entfernen.

## Revendications

1. Un procédé pour la déshydrogénation de l'éthylbenzène en styrène, ce procédé comprenant les étapes de :
faire passer un courant d'alimentation d'éthylbenzène sur un catalyseur de déshydrogénation dans un réacteur de déshydrogénation pour former un courant de produit de styrène;
éliminer de ce réacteur un courant de gaz d'échappement contenant des produits secondaires comprenant l'hydrogène, de la vapeur d'éthylbenzène, des vapeurs de styrène, de benzène et de toluène, CO, CO₂ et de la vapeur
d'eau injecter dans ce courant de gaz d'échappement un inhibiteur de polymérisation de styrène; et,
comprimer ce courant de gaz d'échappement pour un traitement ultérieur.

2. Le procédé selon la revendication 1 dans lequel cet inhibiteur de polymérisation est un composé de phénylènediamine.

3. Un système pour la déshydrogénation de l'éthylbenzène en styrène, ce système comprenant:
un réacteur de déshydrogénation catalytique adapté pour la déshydrogénation de l'éthylbenzène en styrène
un conduit d'amenée de courant d'alimentation d'éthylbenzène raccordé à ce réacteur;
un sous-système d'élimination de gaz d'échappement raccordé à ce réacteur et conçu pour enlever de ce réacteur des produits secondaires de gaz d'échappement provenant de la déshydrogénation de l'éthylbenzène;
un compresseur de gaz d'échappement raccordé à ce sous-système d'élimination et conçu pour recevoir le gaz d'échappement de celui-ci; et,
un sous-système inhibiteur de la polymérisation entre ce sous-système d'élimination et ce compresseur conçu pour injecter un inhibiteur de polymérisation dans un courant d'échappement depuis ce réacteur.
